# FASCICULE DE BREVET EUROPEEN

(11) **EP 3 487 453 B1**
(45) Date de publication et mention de la délivrance du brevet: **28.10.2020**
(21) Numéro de dépôt: 17746425.2
(22) Date de dépôt: 20.07.2017
(51) Int. Cl.: A61F 2/30, A61B 5/00, A61F 2/38

(54) **IMPLANT PROTHETIQUE ET PROCEDE DE FABRICATION D'UN TEL IMPLANT**
PROTHETISCHES IMPLANTAT UND VERFAHREN ZUR HERSTELLUNG SOLCH EINES IMPLANTATS
PROSTHETIC IMPLANT AND METHOD FOR THE PRODUCTION OF SUCH AN IMPLANT

(30) Priorité: 20.07.2016 FR 1656918
(43) Date de publication de la demande: 29.05.2019
(73) Titulaire: Centre National de la Recherche Scientifique, 75016 Paris (FR); Université de Montpellier, 34090 Montpellier (FR); Bonetag, 66000 Perpignan (FR)
(72) Inventeur: VENA, Arnaud, 34270 Saint Mathieu de Tréviers (FR); SORLI, Brice, 34530 Montagnac (FR); CAHUZAC, Stéphan, 34560 Villeveyrac (FR); NAUDI, Stéphane, 66000 Perpignan (FR)
(74) Mandataire: IPAZ
(86) Numéro de dépôt international: PCT/EP2017/068422
(87) Numéro de publication internationale: WO 2018/015513

(56) Documents cités:
- WO-A1-2015/024821
- US-A1- 2005 010 299
- US-A1- 2006 047 283
- US-A1- 2015 238 691

## Description

### Domaine de l'invention

L'invention appartient au domaine des prothèses, de préférence celui des prothèses articulaires, aussi appelées implants prothétiques, par exemple pour hanche, genou, rachis ou coude, qui sont des dispositifs artificiels destinés à suppléer à un organe ou à un membre ou à le remplacer.

L'invention concerne en particulier le domaine des prothèses équipées d'un moyen de communication.

### Art antérieur

On connaît le document US 2005/010299 A1 qui décrit un système d'endoprothèse articulaire qui comprend deux composants prothétiques à fixer sur deux os d'une articulation humaine. Un capteur et des composants électroniques sont fixés à l'un des composants prothétiques de manière à pouvoir conserver un enregistrement du nombre de fois où le patient déplace l'articulation prothétique d'une manière particulière.

On connaît aussi des prothèses comportant une radio-étiquette associée à des capteurs de grandeurs physiques, par exemple de température. De telles prothèses permettent, par exemple, un suivi dans le temps de la température.

La radio-étiquette est installée de manière permanente au contact de la prothèse, ce qui engendre de nombreux inconvénients. Le remplacement de radio-étiquettes défectueuses peut être un problème. La mise en place de la prothèse sans endommager la radio-étiquette qui est fragile est un problème.

Un but de l'invention est de proposer un implant prothétique :
- dont le remplacement de la radio-étiquette est plus aisé que le remplacement de la radio-étiquette dans un implant prothétique selon l'art antérieur, et/ou
- dont la radio-étiquette n'est pas endommagée lors de la mise en place de la prothèse.

### Exposé de l'invention

Selon un premier aspect de l'invention, on atteint un objectif précité avec un implant prothétique comprenant une cavité débouchant sur une face extérieure de l'implant prothétique, la cavité formant un logement pour recevoir :
- un circuit imprimé comportant une radio-étiquette, la radio-étiquette comprenant une antenne associée à une puce électronique contenant un identifiant et des données complémentaires,
- un capot (couvercle) fermant au moins en partie la cavité lorsqu'il est positionné sur l'implant dans une position dite fermée.

Selon le premier aspect de l'invention, le capot et le circuit imprimé sont reçus de manière amovible dans la cavité.

Le circuit imprimé, aussi appelé PCB (pour l'anglais « Printed Circuit Board »), peut comprendre un support, de type diélectrique, aussi appelé substrat. Le circuit imprimé peut être disposé, dans la position fermée du capot, au sein de la cavité.

Le support peut être entourée sur une, ou plusieurs, de ses faces, de l'antenne.

Les données complémentaires peuvent provenir d'enregistrements réalisés à partir de données captées par des capteurs de grandeurs physiques. Les capteurs de grandeurs physiques peuvent par exemple être des capteurs de force ou de température.

Le remplacement de la radio-étiquette est de préférence plus aisé que le remplacement de la radio-étiquette dans un implant prothétique selon l'art antérieur.

La radio-étiquette n'est, de préférence, pas endommagée lors de la mise en place de l'implant prothétique.

Le capot forme, de préférence, une frontière entre la cavité qui est intérieure à l'implant et une partie extérieure à l'implant.

Le capot peut avantageusement fermer totalement la cavité.

La communication avec la radio-étiquette peut être réalisée en utilisant le protocole défini par le standard « EPC Gen 2 » en bande UHF (norme ISO/IEC 18000-6c). Ce protocole permet la lecture/écriture de données.

En bande HF, les normes utilisées sont l'ISO/IEC 14443 et ISO/IEC 15693.

La communication peut être bidirectionnelle.

Avantageusement, l'implant prothétique peut comprendre un capteur capacitif de proximité qui peut être disposé sur le circuit imprimé et peut être raccordé au circuit imprimé. Le principe de détection peut de préférence être basé sur une mesure diélectrique des tissus biologiques à proximité du capot. Un descellement peut être détecté si une variation relative des propriétés diélectriques (différence de constante diélectrique entre l'os, le ciment, le liquide biologique) est détectée.

Selon une possibilité, le capot peut présenter une région séparant la cavité de l'extérieur de l'implant prothétique, ladite région pouvant être conçue pour transmettre une force exercée d'un côté extérieur de ladite région à un côté intérieur de ladite région.

La région peut être réalisée d'un seul bloc avec le capot, l'épaisseur pouvant être amincie au niveau, de préférence sur toute la surface, de ladite région.

De préférence, le capot présente une fenêtre disposée sur une surface séparant la cavité de l'extérieur de l'implant prothétique, la fenêtre étant agencée pour recevoir une membrane déformable, la membrane déformable étant agencée pour transmettre une force exercée sur la membrane à un capteur de force disposé sur le circuit imprimé et raccordé au circuit imprimé. Le capot peut être aminci pour favoriser les déformations de la membrane.

Le capteur de force peut être de type piézorésistif et un élément de transmission de force peut être fonctionnellement disposé entre la membrane et le capteur de force, l'élément de transmission pouvant être agencé pour transmettre une force exercée sur ladite membrane au capteur de force. La résistance d'un matériau piézorésistif peut varier en fonction des contraintes mécaniques.

L'élément de transmission de force peut être un corps déformable, une pièce mobile, un ressort, une lame métallique.

Le capteur de force peut être de type capacitif et un élément de réaction peut être disposé entre la membrane et le capteur de force, l'élément de réaction peut être solidarisé à la membrane, du côté d'une face de la membrane tournée vers la cavité, l'élément de réaction pouvant être agencé pour être déplacé lorsqu'une force est exercée sur ladite membrane.

L'élément de réaction peut être un insert métallique. L'élément de réaction peut être clipsé ou collé à la membrane. L'élément de réaction et des électrodes du capteur de force de type capacitif forment une capacité. Cette capacité peut permettre de détecter une déformation de la membrane. La capacité peut varier lorsque l'élément de réaction est déplacé.

L'élément de réaction peut être un insert diélectrique à permittivité élevée, par exemple en céramique.

Avantageusement, le capteur de force peut être de type piézoélectrique et un corps souple piézoélectrique peut être disposé entre la membrane et le capteur de force, le corps souple peut être solidarisé à la membrane du côté d'une face de la membrane tournée vers la cavité, le corps souple pouvant être agencé pour être déformé lorsqu'une force est exercée sur ladite membrane.

Le corps souple peut être collé sur la membrane déformable. La déformation de la membrane peut induire une déformation mécanique du matériau piézoélectrique et peut produire des charges électriques.

De préférence, l'antenne peut être disposée sur le circuit imprimé du côté de la face extérieure de l'implant prothétique lorsque le circuit imprimé est reçu dans la cavité. De préférence, le rayonnement peut ne pas être confiné dans la cavité. Le rayonnement peut se propager à l'extérieur de l'implant prothétique.

De préférence, au moins une face de l'antenne est disposée en regard d'une face de l'implant métallique. De préférence, la face de l'antenne disposée en regard de la face de l'implant métallique est à une distance inférieure à un seuil, dit seuil de couplage. Ledit seuil de couplage est déterminé pour permettre un couplage entre ladite face de l'antenne et ladite face de l'implant métallique lorsque l'antenne est positionnée dans le logement.

Le circuit imprimé peut être solidarisé au capot. Le circuit imprimé peut par exemple être scellé au capot, par exemple au moyen d'ergots disposés entre le circuit imprimé et le capot. Le capot peut par exemple être surmoulé sur le circuit imprimé. La tenue mécanique dans le temps du l'implant et/ou la résistance aux chocs lors de la pose de l'implant peuvent, par exemple, ainsi, être améliorées.

Le circuit imprimé peut être monté amovible par rapport au capot. Une résine peut être injectée dans le capot pour maintenir le circuit imprimé sur le capot. Une telle résine, une fois solidifiée, peut réaliser un matériau piézorésistif.

Le capot peut être conçu pour recevoir un tiroir à coulissement présentant un logement pour recevoir le circuit imprimé.

Le capot peut présenter un matériau biocompatible sur tout ou partie de la surface apparente du capot lorsque le capot est positionné dans la position fermée.

Le matériau biocompatible peut être du polyéthylène PE, du PEEK, du polypropylène PP, du polysulphone PS, du PTFE. Le polyéthylène PE est utilisé dans les prothèses. Un autre matériau biocompatible peut être du parylène.

Le capot peut être maintenu dans une position fermée par une résine injectée sur au moins une partie dudit capot, en particulier à la frontière formée par le capot et la surface extérieure sur laquelle débouche la cavité. La résine permet d'assurer l'étanchéité de la cavité lorsque le capot est en position fermée. La radio-étiquette est ainsi plus protégée. En particulier, la résine peut être biocompatible. La norme de la résine peut être la norme USP Class VI ou la norme ISO 10993-5.

La cavité peut être une rainure agencée pour coopérer avec le capot.

La cavité et le capot peuvent former une queue d'aronde. La rainure peut être formée par la cavité. Le tenon peut être formé par le capot.

L'implant selon l'invention peut comprendre un support agencé dans la cavité prévue pour recevoir le circuit imprimé et pour former avec le capot un boitier étanche. La radio-étiquette est ainsi plus protégée.

Le support peut avoir une épaisseur au moins inférieure à la moitié de l'épaisseur de la cavité.

Le support peut comprendre un logement ayant la forme du circuit imprimé.

Le support peut être fabriqué dans un matériau biocompatible, en particulier un matériau étanche, par exemple les métaux et alliages métalliques comme le titane, l'acier inoxydable 316L.

Le support peut être fabriqué en polymère, en métal ou en céramique comme la zircone.

Avantageusement, au moins une face de l'antenne peut être disposée en regard d'au moins une face de l'implant prothétique, ladite face de l'implant prothétique étant métallique, de manière à réaliser un couplage inductif et/ou capacitif entre ladite au moins une face de l'antenne et ladite au moins une face de l'implant prothétique, de préférence à une distance inférieure à 2 mm. L'implant prothétique peut être entièrement métallique. On utilise ainsi la capacité naturelle de rayonnement de l'implant prothétique pour amplifier le rayonnement de l'antenne.

Selon un autre aspect de l'invention, il est proposé un procédé de fabrication d'un implant prothétique conforme au premier aspect de l'invention.

Le procédé de fabrication peut comporter une fabrication d'une prothèse pleine et une étape d'usinage de la prothèse pleine pour former la cavité de l'implant prothétique.

Alternativement, ou en complément de ce qui précède, le procédé de fabrication peut être directement réalisé à partir d'un moule.

### Description des figures

D'autres particularités et avantages de l'invention apparaîtront à la lecture de la description détaillée de mises en œuvre et de modes de réalisation nullement limitatifs, au regard de figures annexées sur lesquelles :
- la figure 1 est une illustration en perspective d'un mode de réalisation d'un implant prothétique selon l'invention ;
- la figure 2 est une autre illustration en perspective du mode de réalisation de l'implant prothétique de la figure 1 ;
- la figure 3 est une illustration en perspective d'un autre mode de réalisation de l'implant prothétique selon l'invention.

### Description détaillée

Ces modes de réalisation n'étant nullement limitatifs, on pourra notamment réaliser des variantes de l'invention ne comprenant qu'une sélection de caractéristiques décrites par la suite, telles que décrites ou généralisées, isolées des autres caractéristiques décrites, si cette sélection de caractéristiques est suffisante pour conférer un avantage technique ou pour différencier l'invention par rapport à l'état de la technique.

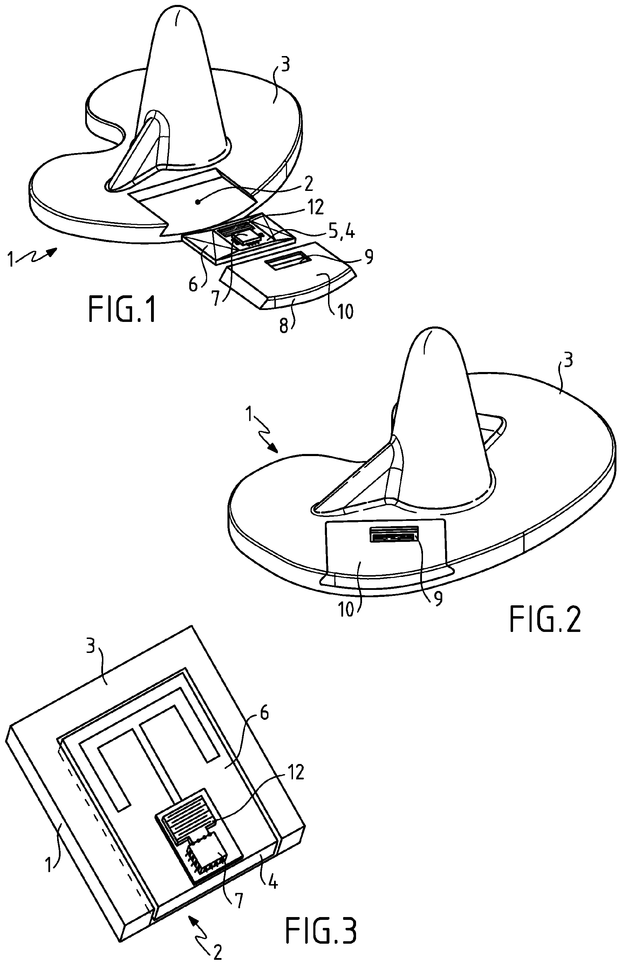

Sur la figure 1, il est illustré un implant prothétique 1, de préférence métallique, selon l'invention. L'implant prothétique 1 est un plateau tibial. L'implant prothétique 1 comprend une cavité 2 débouchant sur une face extérieure 3 de l'implant prothétique 1.

Le procédé de fabrication de l'implant prothétique 1 peut comporter une fabrication d'une prothèse pleine, de préférence métallique, et une étape d'usinage de la prothèse pleine pour former la cavité 2 de l'implant prothétique 1.

Alternativement, ou en complément de ce qui précède, le procédé de fabrication de l'implant prothétique 1 peut être directement réalisé à partir d'un moule pour prothèse, de préférence métallique.

La cavité 2 forme un logement et peut recevoir :
- un circuit imprimé 4 comportant une radio-étiquette 5. La radio-étiquette 5 comprend une antenne 6 associée à une puce électronique 7 contenant un identifiant et des données complémentaires ;
- un capot 8 fermant au moins en partie la cavité lorsqu'il est positionné sur l'implant (voir figure 2) dans une position dite fermée.

Dans un mode de réalisation, la cavité 2 représente 7,2 % du volume de l'implant prothétique. Bien entendu, ce ratio peut varier en fonction de la taille de l'implant prothétique.

La communication avec la radio-étiquette 5 peut être réalisée en utilisant le protocole défini par le standard « EPC Gen 2 » en bande UHF (norme ISO/IEC 18000-6c). Ce protocole permet la lecture/écriture de données.

En bande HF, les normes utilisées sont l'ISO/IEC 14443 et ISO/IEC 15693.

La communication peut être bidirectionnelle.

Le capot 8 peut protéger la cavité 2.

Le capot 8 peut protéger le circuit imprimé 4 lorsque le circuit imprimé 4 est disposé dans la cavité 2.

Le capot 8 et le circuit imprimé 4 sont reçus de manière amovible dans la cavité 2.

Le capot 8 forme une frontière entre la cavité 2 qui est intérieure à l'implant prothétique 1 et une partie extérieure à l'implant.

Le capot 8 peut avantageusement fermer totalement la cavité 2, ce qui n'est pas illustré par les figures.

L'implant prothétique peut comprendre, mais ce n'est pas illustré par les figures, un capteur capacitif de proximité. Le capteur capacitif peut être disposé sur le circuit imprimé et peut être raccordé au circuit imprimé. Le principe de détection peut être basé sur une mesure diélectrique des tissus biologiques à proximité du capot. Un descellement peut être détecté si une variation relative des propriétés diélectriques (différence de constante diélectrique entre l'os, le ciment, le liquide biologique) est détectée.

Comme l'illustrent les figures 1 et 2, le capot 8 présente une fenêtre 9 disposée sur une surface 10 séparant la cavité de l'extérieur de l'implant prothétique. La fenêtre 9 est agencée pour recevoir une membrane déformable (non représentée). La membrane déformable est agencée pour transmettre une force exercée sur la membrane à un capteur de force 12 disposé sur le circuit imprimé 4 et raccordé au circuit imprimé 4. Le capot 8 peut être aminci pour favoriser les déformations de la membrane.

Le capteur de force 12 est de type piézorésistif et un élément de transmission de force (non représenté) est fonctionnellement disposé entre la membrane et le capteur de force 12. L'élément de transmission est agencé pour transmettre une force exercée sur la membrane au capteur de force 12.

L'élément de transmission de force peut être un corps déformable. Dans d'autres exemples, l'élément de transmission de force pourrait être une pièce mobile, un ressort, ou une lame métallique.

Dans un exemple non davantage représenté, alternativement ou en complément de ce qui précède, le capteur de force peut être de type capacitif et un élément de réaction (non représenté) peut être disposé entre la membrane et le capteur de force. L'élément de réaction peut être solidarisé à la membrane, du côté d'une face de la membrane tournée vers la cavité 2. L'élément de réaction peut être agencé pour être déplacé lorsqu'une force est exercée sur ladite membrane.

Dans cet exemple non davantage représenté, alternativement ou en complément de ce qui précède, l'élément de réaction peut être un insert métallique (non représenté). L'élément de réaction peut être clipsé ou collé à la membrane. L'élément de réaction et des électrodes du capteur de force de type capacitif forment une capacité. Cette capacité peut permettre de détecter une déformation de la membrane. La capacité peut varier lorsque l'élément de réaction est déplacé.

Selon une alternative non davantage représentée, alternativement ou en complément de ce qui précède, l'élément de réaction peut être un insert diélectrique (non représenté) à permittivité élevée, par exemple en céramique.

Dans un exemple non davantage représenté, alternativement ou en complément de ce qui précède, le capteur de force 12 peut être de type piézoélectrique et un corps souple piézoélectrique (non représenté) peut être disposé entre la membrane et le capteur de force 12. Le corps souple peut être solidarisé à la membrane du côté d'une face (non représentée) de la membrane tournée vers la cavité 2. Le corps souple peut être agencé pour être déformé lorsqu'une force est exercée sur la membrane.

Le corps souple peut être collé sur la membrane déformable. La déformation de la membrane peut induire une déformation mécanique du matériau piézoélectrique et peut produire des charges électriques.

Dans l'exemple représenté sur la figure 1, l'antenne 6 est disposée sur le circuit imprimé 4 du côté de la face extérieure 3 de l'implant prothétique 1 lorsque le circuit imprimé 4 est reçu dans la cavité 2. Le rayonnement de l'antenne 6 peut ne pas être confiné dans la cavité. Le rayonnement de l'antenne 6 peut se propager à l'extérieur de l'implant prothétique 1.

Dans un exemple non représenté, le circuit imprimé 4 est solidarisé au capot 8. Le circuit imprimé 4 peut par exemple être scellé au capot 8, par exemple aux moyens d'ergots disposés entre le circuit imprimé 4 et le capot 8. Le capot 8 peut par exemple être surmoulé sur le circuit imprimé 4.

Dans l'exemple représenté sur la figure 1, le circuit imprimé 4 est monté amovible par rapport au capot 8. Dans cet exemple, le capot 8 est conçu pour recevoir un tiroir à coulissement (non représenté) présentant un logement pour recevoir le circuit imprimé 4.

Le capot 8 peut présenter un matériau biocompatible sur tout ou partie de la surface apparente du capot 8 lorsque le capot est positionné dans la position fermée. Le matériau biocompatible peut être du polyéthylène PE, du PEEK, du polypropylène PP, du polysulphone PS, du PTFE. Dans l'exemple représenté, le matériau biocompatible est du polyéthylène PE. La cavité peut être une rainure agencée pour coopérer avec le capot.

Comme l'illustre la figure 1, la cavité 2 et le capot 8 forment une queue d'aronde. La rainure de la queue d'aronde est formée par la cavité 2. Le tenon de la queue d'aronde est formé par le capot 8.

On va maintenant décrire, en référence à la figure 3, un autre mode de réalisation de l'implant selon l'invention. Les références utilisées pour décrire la figure 1 et la figure 2 sont reprises.

La figure 3 est une vue schématique en perspective de l'implant prothétique 1 au niveau de la cavité 2 adaptée à recevoir le circuit imprimé.

Dans cet exemple, le logement présente deux faces ouvertes sur l'extérieur de l'implant prothétique, les autres faces du logement étant constituées par des faces de l'implant prothétique.

Le circuit imprimé peut avoir une forme correspondant au logement formé dans l'implant.

Le support de circuit peut, par exemple, être réalisé en une résine époxy ou en un composite de résine époxy renforcé de fibre de verre, par exemple de type FR-4, le FR-4 (pour l'anglais « Flame Resistant 4 ») étant utilisé pour la fabrication de circuit imprimé.

En particulier, le circuit imprimé peut présenter une forme parallélépipédique. Les dimensions du parallélépipède peuvent être dans une gamme d'épaisseur allant de 0,1 à 5 mm, dans une gamme de largeur allant de 5 à 35 mm et dans une gamme de longueur allant de 5 à 35 mm.

Le support de circuit imprimé peut être entouré par l'antenne 6, sur tout ou partie de ses faces en regard d'une face de l'implant prothétique. Il est ainsi réalisé un couplage inductif et capacitif entre l'implant prothétique et l'antenne.

Par exemple, chaque distance entre une première face de l'antenne en regard de l'implant prothétique et une deuxième face correspondante de l'implant prothétique en regard de ladite première face de l'antenne peut être inférieure à 2 mm.

Un espace réduit permet de favoriser le couplage des courants de surface de l'antenne formée sur le support de circuit imprimé avec l'implant prothétique métallique.

Un implant prothétique métallique est un bon conducteur qui autorise l'établissement de courants induits qui sont source de rayonnement électromagnétique.

Lorsque des courants induits sont couplés entre l'antenne et l'implant prothétique, l'intensité du champ rayonné est importante.

Il est en outre prévu de disposer les faces du circuit imprimé ne présentant pas d'éléments conducteurs à distance de l'implant prothétique, pour augmenter l'efficacité de rayonnement par les ouvertures.

Sur la figure 3, il est également représenté la puce électronique 7 qui comprend 16 broches.

Deux broches sont électriquement reliées à l'antenne 6. Deux autres broches sont électriquement reliées au capteur de force 12.

La face du circuit imprimé disposée du côté de la face extérieure 3 de l'implant prothétique présente une ouverture en forme de T.

L'antenne enveloppe peut être réalisée en cuivre. Elle peut entourer tout le circuit imprimé, à l'exception des deux faces comprenant des ouvertures.

Bien sûr, l'invention n'est pas limitée aux exemples qui viennent d'être décrits et de nombreux aménagements peuvent être apportés à ces exemples sans sortir du cadre de l'invention. De plus, les différentes caractéristiques, formes, variantes et modes de réalisation de l'invention peuvent être associés les uns avec les autres selon diverses combinaisons dans la mesure où ils ne sont pas incompatibles ou exclusifs les uns des autres.

## Revendications

1. Implant prothétique (1) comprenant une cavité (2) débouchant sur une face extérieure (3) dudit implant prothétique (1), ladite cavité (2) formant logement pour recevoir :
- un circuit imprimé (4) comportant une radio-étiquette (5), ladite radio-étiquette (5) comprenant une antenne (6) associée à une puce électronique (7), **caractérisé en ce que** la puce électronique contient un identifiant et des données complémentaires, et **en ce qu'**un capot (8) ferme au moins en partie ladite cavité (2) lorsqu'il est positionné sur ledit implant (1) dans une position dite fermée, la radio-étiquette (5) et le circuit imprimé (4) étant reçus de manière amovible dans ladite cavité (2).

2. Implant prothétique selon la revendication 1, comprenant un capteur capacitif de proximité disposé sur le circuit imprimé et raccordé au circuit imprimé.

3. Implant prothétique selon la revendication 1 ou 2, dans lequel le capot présente une région séparant la cavité de l'extérieur de l'implant prothétique, ladite région étant conçue pour transmettre une force exercée d'un côté extérieur de ladite région à un côté intérieur de ladite région.

4. Implant prothétique selon la revendication 3, dans lequel la région est réalisée d'un seul bloc avec le capot, l'épaisseur étant amincie au niveau de ladite région.

5. Implant prothétique selon la revendication 1 ou 2, dans lequel le capot présente une fenêtre disposée sur une surface séparant la cavité de l'extérieur de l'implant prothétique, ladite fenêtre étant agencée pour recevoir une membrane déformable et transmettre une force exercée sur ladite membrane à un capteur de force disposé sur le circuit imprimé et raccordé au circuit imprimé.

6. Implant prothétique selon la revendication 5, dans lequel le capteur de force est de type piézorésistif et un élément de transmission de force est disposé entre la membrane et ledit capteur de force, l'élément de transmission étant agencé pour transmettre une force exercée sur ladite membrane au capteur de force.

7. Implant prothétique selon la revendication 5, dans lequel le capteur de force est de type capacitif et un élément de réaction est disposé entre la membrane et ledit capteur de force, ledit élément de réaction étant solidarisé à la membrane, du côté d'une face de la membrane tournée vers la cavité, l'élément de réaction étant agencé pour être déplacé lorsqu'une force est exercée sur ladite membrane.

8. Implant prothétique selon la revendication 5, dans lequel le capteur de force est de type piézoélectrique et un corps souple piézoélectrique est disposé entre la membrane et ledit capteur de force, ledit corps souple étant solidarisé à la membrane du côté d'une face de la membrane tournée vers la cavité, ledit corps souple étant agencé pour être déformé lorsqu'une force est exercée sur ladite membrane.

9. Implant prothétique selon l'une quelconque des revendications précédentes, dans lequel l'antenne est disposée sur le circuit imprimé du côté de la face extérieure de l'implant prothétique lorsque le circuit imprimé est reçu dans la cavité.

10. Implant prothétique selon l'une quelconque des revendications précédentes, dans lequel le circuit imprimé est solidarisé au capot.

11. Implant prothétique selon l'une quelconque des revendications précédentes, dans lequel le circuit imprimé est monté amovible par rapport au capot.

12. Implant prothétique selon la revendication 9, dans lequel le capot est agencé pour recevoir un tiroir à coulissement présentant un logement pour recevoir le circuit imprimé.

13. Implant prothétique selon l'une quelconque des revendications précédentes, dans lequel ledit capot présente un matériau biocompatible sur tout ou partie de la surface apparente dudit capot lorsque le capot est positionné dans la position fermée.

14. Implant prothétique selon l'une quelconque des revendications précédentes, dans lequel le capot est maintenu dans une position fermée par une résine injectée sur au moins une partie dudit capot, en particulier à la frontière formée par le capot et la surface extérieure sur laquelle débouche la cavité.

15. Implant prothétique selon l'une quelconque des revendications précédentes, dans lequel la cavité est une rainure agencée pour coopérer avec le capot.

16. Implant prothétique selon l'une quelconque des revendications précédentes, dans lequel cavité et capot forment une queue d'aronde.

17. Implant prothétique selon l'une quelconque des revendications précédentes, dans lequel au moins une face de l'antenne est disposée en regard d'au moins une face de l'implant prothétique, ladite face de l'implant prothétique étant métallique, de manière à réaliser un couplage inductif et/ou capacitif entre ladite au moins une face de l'antenne et ladite au moins une face de l'implant prothétique, de préférence à une distance inférieure à 2 mm.

18. Procédé de fabrication d'un implant prothétique selon l'une quelconque des revendications 1 à 17, comportant une fabrication d'une prothèse pleine et une étape d'usinage de ladite prothèse pleine pour former la cavité de l'implant prothétique.

19. Procédé de fabrication d'un implant prothétique selon l'une quelconque des revendications 1 à 17, dans lequel l'implant prothétique est directement réalisé à partir d'un moule.

## Patentansprüche

1. Prothetisches Implantat (1), einen Hohlraum (2) umfassend, welcher auf einer Außenseite (3) des prothetischen Implantats (1) mündet, wobei der Hohlraum (2) eine Aufnahme bildet zur Aufnahme von:
- einer Leiterplatte (4), beinhaltend einen RFID-Tag (5), wobei der RFID-Tag (5) eine einem Chip (7) zugeordnete Antenne (6) umfasst, **dadurch gekennzeichnet, dass** der Chip einen Identifikator und Zusatzdaten enthält und dadurch, dass eine Klappe (8) zumindest teilweise den Hohlraum (2) verschließt, wenn sie auf dem Implantat (1) in einer sogenannten geschlossenen Position gelagert ist,
wobei der RFID-Tag (5) und die Leiterplatte (4) lösbar in dem Hohlraum (2) aufgenommen sind.

2. Prothetisches Implantat nach Anspruch 1, einen kapazitiven Näherungssensor umfassend, welcher auf der Leiterplatte gelagert und mit der Leiterplatte angeschlossen ist.

3. Prothetisches Implantat nach Anspruch 1 oder 2, bei welchem die Klappe einen Bereich aufweist, welcher den Hohlraum von der Außenseite des prothetischen Implantats trennt, wobei der Bereich dazu ausgebildet ist, eine von einer Außenseite des Bereichs aus wirkende Kraft auf eine Innenseite des Bereichs zu übertragen.

4. Prothetisches Implantat nach Anspruch 3, bei welchem der Bereich einstückig mit der Klappe ausgeführt ist, wobei die Dicke an dem Bereich abnimmt.

5. Prothetisches Implantat nach Anspruch 1 oder 2, bei welchem die Klappe ein Fenster aufweist, welches auf einer den Hohlraum von dem Äußeren des prothetischen Implantats trennenden Oberfläche gelagert ist, wobei das Fenster zur Aufnahme einer verformbaren Membran und zur Übertragung einer auf der Membran wirkenden Kraft an einem Kraftsensor angeordnet ist, welcher auf der Leiterplatte gelagert und mit der Leiterplatte angeschlossen ist.

6. Prothetisches Implantat nach Anspruch 5, bei welchem der Kraftsensor piezoresistiv ist und ein Kraftübertragungselement zwischen der Membran und dem Kraftsensor gelagert ist, wobei das Kraftübertragungselement dazu angeordnet ist, eine auf der Membran wirkende Kraft auf den Kraftsensor zu übertragen.

7. Prothetisches Implantat nach Anspruch 5, bei welchem der Kraftsensor kapazitiv ist und ein Reaktionselement zwischen der Membran und dem Kraftsensor gelagert ist, wobei das Reaktionselement mit der Membran seitens einer dem Hohlraum zugewandten Membranseite verbunden ist, wobei das Reaktionselement bewegbar angeordnet ist, wenn die Membran mit einer Kraft beaufschlagt wird.

8. Prothetisches Implantat nach Anspruch 5, bei welchem der Kraftsensor piezoelektrisch ist und ein piezoelektrischer, weicher Körper zwischen der Membran und dem Kraftsensor gelagert ist, wobei der weiche Körper mit der Membran seitens einer dem Hohlraum zugewandten Membranseite verbunden ist, wobei der weiche Körper verformbar angeordnet ist, wenn die Membran mit einer Kraft beaufschlagt wird.

9. Prothetisches Implantat nach einem der vorhergehenden Ansprüche, bei welchem die Antenne auf der Leiterplatte seitens der Außenseite des prothetischen Implantats gelagert ist, wenn die Leiterplatte im Hohlraum aufgenommen ist.

10. Prothetisches Implantat nach einem der vorhergehenden Ansprüche, bei welchem die Leiterplatte mit der Klappe verbunden ist.

11. Prothetisches Implantat nach einem der vorhergehenden Ansprüche, bei welchem die Leiterplatte lösbar gegenüber der Klappe montiert ist.

12. Prothetisches Implantat nach Anspruch 9, bei welchem die Klappe dazu angeordnet ist, einen Gleitschieber aufzunehmen, welcher eine Aufnahme zur Aufnahme der Leiterplatte aufweist.

13. Prothetisches Implantat nach einem der vorhergehenden Ansprüche, bei welchem die Klappe ein biokompatibles Material auf der gesamten oder einem Teil der sichtbaren Oberfläche der Klappe aufweist, wenn sich die Klappe in der geschlossenen Position befindet.

14. Prothetisches Implantat nach einem der vorhergehenden Ansprüche, bei welchem die Klappe in einer geschlossenen Position durch ein Harz gehalten wird, das mindestens auf einem Teil der Klappe, insbesondere an der Grenze injiziert wird, welche durch die Klappe und der Außenfläche gebildet wird, auf welcher der Hohlraum mündet.

15. Prothetisches Implantat nach einem der vorhergehenden Ansprüche, bei welchem der Hohlraum eine Nut ist, welche zur Zusammenwirkung mit der Klappe angeordnet ist.

16. Prothetisches Implantat nach einem der vorhergehenden Ansprüche, bei welchem Hohlraum und Klappe einen Schwalbenschwanz bilden.

17. Prothetisches Implantat nach einem der vorhergehenden Ansprüche, bei welchem mindestens eine Seite der Antenne mindestens einer Seite des prothetischen Implantats zugewandt ist, wobei die Seite des prothetischen Implantats derart metallisch ist, sodass eine induktive und/oder kapazitive Koppelung zwischen der mindestens einen Seite der Antenne und der mindestens einen Seite des prothetischen Implantats bevorzugt in einem Abstand kleiner 2 mm ausgeführt wird.

18. Verfahren zur Herstellung eines prothetischen Implantats nach einem der Ansprüche 1 bis 17, beinhaltend eine Herstellung einer Vollprothese und einen Verarbeitungsschritt der Vollprothese zur Ausbildung des Hohlraums des prothetischen Implantats.

19. Verfahren zur Herstellung eines prothetischen Implantats nach einem der Ansprüche 1 bis 17, bei welchem das prothetische Implantat direkt anhand einer Form ausgeführt wird.

## Claims

1. Prosthetic implant (1) comprising a cavity (2) opening onto an outer face (3) of said prosthetic implant (1), said cavity (2) forming a housing for receiving:
- a printed circuit board (4) including an RFID tag (5), said RFID tag (5) comprising an antenna (6) connected to an electronic chip (7), **characterized in that** the electronic chip contains an identifier and additional data, and **in that** a cover (8) closes at least in part said cavity (2) when it is positioned on said implant (1) in a position called closed position,
the RFID tag (5) and the printed circuit board (4) being received removably in the cavity (2).

2. Prosthetic implant according to claim 1, comprising a capacitive proximity sensor arranged on the printed circuit board and connected to the printed circuit board.

3. Prosthetic implant according to claim 1 or 2, in which the cover has a region separating the cavity from the outside of the prosthetic implant, said region being designed to transmit a force exerted from an outer side of said region to an inner side of said region.

4. Prosthetic implant according to claim 3, in which the region is produced in a single piece with the cover, the thickness being reduced at the level of said region.

5. Prosthetic implant according to claim 1 or 2, in which the cover has a window arranged on a surface separating the cavity from the outside of the prosthetic implant, said window being arranged in order to receive a deformable membrane and to transmit a force exerted on said membrane to a force sensor arranged on the printed circuit board and connected to the printed circuit board.

6. Prosthetic implant according to claim 5, in which the force sensor is of the piezoresistive type and a force-transmission element is arranged between the membrane and said force sensor, the transmission element being arranged in order to transmit a force exerted on said membrane to the force sensor.

7. Prosthetic implant according to claim 5, in which the force sensor is of the capacitive type and a reaction element is arranged between the membrane and said force sensor, said reaction element being firmly fastened to the membrane, on the side of a face of the membrane turned towards the cavity, the reaction element being arranged in order to be displaced when a force is exerted on said membrane.

8. Prosthetic implant according to claim 5, in which the force sensor is of the piezoelectric type and a flexible piezoelectric body is arranged between the membrane and said force sensor, said flexible body being firmly fastened to the membrane, on the side of a face of the membrane turned towards the cavity, said flexible body being arranged in order to be deformed when a force is exerted on said membrane.

9. Prosthetic implant according to any one of the preceding claims, in which the antenna is arranged on the printed circuit board on the side of the outer face of the prosthetic implant when the printed circuit board is received in the cavity.

10. Prosthetic implant according to any one of the preceding claims, in which the printed circuit board is firmly fastened to the cover.

11. Prosthetic implant according to any one of the preceding claims, in which the printed circuit board is mounted removably with respect to the cover.

12. Prosthetic implant according to claim 9, in which the cover is arranged in order to receive a sliding drawer having a housing for receiving the printed circuit board.

13. Prosthetic implant according to any one of the preceding claims, in which said cover has a biocompatible material over all or part of the exposed surface of said cover when the cover is positioned in the closed position.

14. Prosthetic implant according to any one of the preceding claims, in which the cover is held in a closed position by a resin injected over at least a part of said cover, in particular at the boundary formed by the cover and the outer surface onto which the cavity opens.

15. Prosthetic implant according to any one of the preceding claims, in which the cavity is a groove arranged in order to co-operate with the cover.

16. Prosthetic implant according to any one of the preceding claims, in which cavity and cover form a dovetail.

17. Prosthetic implant according to any one of the preceding claims, in which at least one face of the antenna is arranged opposite at least one face of the prosthetic implant, said face of the prosthetic implant being made of metal, so as to produce an inductive and/or capacitive coupling between said at least one face of the antenna and said at least one face of the prosthetic implant, preferably at a distance less than 2 mm.

18. Method for the production of a prosthetic implant according to any one of claims 1 to 17 comprising production of a solid prosthesis and a step of machining said solid prosthesis in order to form the cavity of the prosthetic implant.

19. Method for the production of a prosthetic implant according to any one of claims 1 to 17, in which the prosthetic implant is produced directly from a mould.
